# FASCICULE DE BREVET EUROPEEN

(11) **EP 2 775 859 B1**
(45) Date de publication et mention de la délivrance du brevet: **18.01.2017**
(21) Numéro de dépôt: 12795522.7
(22) Date de dépôt: 12.11.2012
(51) Int. Cl.: A61K 31/56, A61K 36/21, A61K 31/575, A23L 33/11, A23L 33/105, A23K 20/168, A23K 20/184

(54) **PHYTOECDYSONES POUR LEUR UTILISATION DANS LA STABILISATION DU POIDS CHEZ LES MAMIFÈRES OBÈSES APRÈS UN RÉGIME AMAIGRISSANT**
PHYTOECDYDONEN ZUR STABILISIERUNG DES GEWICHTS BEI FETTLEIBIGEN SÄUGETIEREN NACH EINER HYPOKALORISCHEN DIET
PHYTOECDYSONES FOR STABILIZING WEIGHT IN OBESE PATIENTS AFTER AN HYPOCALORIC DIET

(30) Priorité: 10.11.2011 FR 1160280
(43) Date de publication de la demande: 17.09.2014
(73) Titulaire: Biophytis, 75001 Paris (FR); Université Pierre et Marie Curie, 75005 Paris (FR)
(72) Inventeur: LAFONT, René, F-75016 Paris (FR); CLEMENT, Karine, F-75003 Paris (FR); RIZKALLA, Salwa, F-94150 Rungis (FR); VEILLET, Stanislas, F-91600 Savigny Sur Orge (FR); FOUCAULT, Anne-Sophie, F-75013 Paris (FR); DIOH, Waly, F-91220 Bretigny Sur Orge (FR)
(74) Mandataire: Ipside
(86) Numéro de dépôt international: PCT/FR2012/052600
(87) Numéro de publication internationale: WO 2013/068704

(56) Documents cités:
- FR-A1- 2 924 346
- GB-A- 2 420 066
- FOUCAULT ANNE-SOPHIE ET AL: "Quinoa extract enriched in 20-hydroxyecdysone protects mice from diet-induced obesity and modulates adipokines expression.", OBESITY (SILVER SPRING, MD.) FEB 2012 LNKD- DOI:10.1038/OBY.2011.257 PUBMED:21869758, vol. 20, no. 2, 25 août 2011 (2011-08-25), pages 270-277, XP009159207, ISSN: 1930-7381
- KIZELSZTEIN PABLO ET AL: "20-Hydroxyecdysone decreases weight and hyperglycemia in a diet-induced obesity mice model.", AMERICAN JOURNAL OF PHYSIOLOGY. ENDOCRINOLOGY AND METABOLISM MAR 2009 LNKD- PUBMED:19126784, vol. 296, no. 3, mars 2009 (2009-03), pages E433-E439, XP002675659, ISSN: 0193-1849
- ZHU N ET AL: "Ecdysteroids of quinoa seeds (Chenopodium quinoa Willd.)", JOURNAL OF AGRICULTURAL AND FOOD CHEMISTRY, AMERICAN CHEMICAL SOCIETY, US, vol. 49, 1 janvier 2001 (2001-01-01), pages 2576-2578, XP002483876, ISSN: 0021-8561, DOI: 10.1021/JF0014462 [extrait le 2001-04-13]
- KUMPUN S ET AL: "Ecdysteroids from Chenopodium quinoa Willd., an ancient Andean crop of high nutritional value", FOOD CHEMISTRY, ELSEVIER LTD, NL, vol. 125, no. 4, 15 avril 2011 (2011-04-15), pages 1226-1234, XP027504487, ISSN: 0308-8146, DOI: 10.1016/J.FOODCHEM.2010.10.039 [extrait le 2010-11-17]

## Description

### Domaine technique

L'invention concerne les phytoecdysones, apportées pures ou contenues dans un extrait, pour leur utilisation dans la stabilisation du poids après un régime amaigrissant.

Plus particulièrement, l'invention permet d'éviter la reprise de poids des mammifères obèses préalablement soumis à un régime amaigrissant.

### Etat de la technique

La surcharge pondérale et l'obésité sont de nos jours des états physiopathologiques dont la prévalence augmente régulièrement dans le monde entier. En Europe et aux USA, un individu est considéré en surcharge pondérale dès lors que son indice de masse corporelle (IMC) est supérieur à 25. Il est considéré comme obèse dès que son IMC est supérieur à 30.

Ce désordre physiologique peut être à l'origine de nombreuses complications de santé. Par exemple, le syndrome métabolique est une perturbation physiologique le plus souvent liée au surpoids. On reconnaît qu'une personne est atteinte du syndrome métabolique lorsqu'elle présente au moins trois des cinq signes cliniques associés à cet état, à savoir une obésité viscérale ou abdominale, une hypertriglycéridémie, une dyslipidémie athérogène, une hypertension et une hyperglycémie (Isomaa et al., 2001). L'obésité augmente par ailleurs en elle-même les risques de développer un diabète de type II, ou diabète gras, et des maladies cardio-vasculaires (Rexrode et al., 1998).

Une méthode pour qu'un mammifère obèse perde du poids et de la masse grasse est de suivre un régime hypocalorique, éventuellement associé à des médicaments et/ou compléments alimentaires, ainsi qu'à une activité physique régulière. Cependant, après une première phase de perte de poids, il est fréquent d'observer une reprise de poids chez les individus soumis au régime hypocalorique (Ulen et al., 2008). Ce phénomène est connu sous le nom d'« effet rebond ».

De nombreux compléments alimentaires ou aliments fonctionnels ont été développés pour favoriser la perte de poids chez le mammifère en surpoids (Saper et al., 2004) et prévenir le développement du diabète (McWorther, 2001) et des maladies cardiovasculaires. La plupart des produits alimentaires actuellement sur le marché font preuve d'une efficacité insuffisante pour agir sur l'obésité abdominale et, dans certains cas, ils se sont révélés être toxiques (Pittler et al., 2005). Par ailleurs, ils ne présentent pas d'efficacité à prévenir l'effet rebond après une certaine durée de régime alimentaire hypocalorique.

Il est donc nécessaire d'identifier de nouvelles molécules naturelles, déjà présentes dans l'alimentation des mammifères, pour développer des ingrédients et des aliments fonctionnels non toxiques et efficaces à long terme sur la masse grasse.

Les phytoecdysones sont des ecdystéroïdes d'origine végétale. Il s'agit de molécules naturelles de la famille des triterpènes, relativement abondantes dans le règne végétal où elles sont présentes chez 5% des plantes sauvages (Bàthori et Pongrács, 2005).

Comme décrit dans le brevet FR2924346 au nom de la Demanderesse, les phytoecdysones, en particulier la 20-hydroxyecdysone, sont connues pour diminuer l'augmentation de la masse grasse chez les mammifères soumis à un régime hypercalorique obésifiant.

En outre, ces molécules présentent des propriétés antioxydantes (Kuzmenko et al., 2001) et sont dépourvues de toxicité (Ogawa et al., 1974).

### Exposé de l'invention

Les inventeurs ont découvert que l'ingestion de phytoecdysones, par des mammifères obèses permet de diminuer le phénomène de reprise de poids suite à une première phase de perte de poids grâce à un régime hypocalorique.

Plus précisément, les individus soumis à un traitement par des phytoecdysones stabilisent leur poids, voire continuent à perdre du poids, au cours d'une phase dite de stabilisation, durant laquelle un groupe témoin reprend du poids.

Par ailleurs, durant cette phase de stabilisation, les individus soumis à un traitement par des phytoecdysones présentent une taille adipocytaire plus faible que le groupe témoin. Ils présentent en outre une insulinémie plus faible et une meilleure sensibilité à l'insuline que le groupe témoin.

L'invention propose donc de mettre en oeuvre des phytoecdysones, particulièrement la 20-hydroxyecdysone, pour éviter la reprise de poids chez les mammifères obèses soumis à un régime hypocalorique amaigrissant.

Préférentiellement, les phytoecdysones sont également utilisées pour stabiliser, voire continuer à réduire, le diamètre adipocytaire pendant la phase de stabilisation après un régime hypocalorique amaigrissant.

Préférentiellement, les phytoecdysones sont également utilisées pour stabiliser la sensibilité à l'insuline améliorée par un régime hypocalorique amaigrissant préalable. Outre leur effet dans le traitement de l'obésité, les phytoecdysones présentent donc un intérêt dans le traitement du diabète, notamment du diabète de type II.

Les phytoecdysones utilisées peuvent être obtenues par extraction, à partir de plantes. On peut également utiliser des ecdysones préparées par hémisynthèse.

Les phytoecdysones sont préférentiellement choisies parmi la 20-hydroxyecdysone, la makistérone A, la 24-épi-makistérone A, la 24(28)-déhydro-makistérone A, la 20,26-dihydroxyecdysone et les mélanges de deux ou plus de ces composés.

Les phytoecdysones peuvent être apportées pures, ou sous forme d'extraits de plantes plus ou moins enrichis. Avantageusement, les phytoecdysones mises en oeuvre selon l'invention se présentent sous la forme d'un extrait de plantes enrichi en phytoecdysones, ledit extrait contenant au moins 1% en masse de phytoecdysones. Préférentiellement, l'extrait contient entre 1% et 7% de phytoecdysones, plus préférentiellement entre 1.5% et 3% et encore plus préférentiellement 2% en masse de phytoecdysones.

Les plantes alimentaires à partir desquelles sont réalisés les extraits selon l'invention sont avantageusement choisies parmi les Chénopodiacées, en particulier le quinoa et les épinards (Findeisen, 2004). Des plantes médicinales peuvent également être utilisées pour développer des extraits riches en phytoecdysones.

Préférentiellement, un extrait de plantes enrichi en phytoecdysones selon l'invention provient d'un extrait de quinoa. En effet, le quinoa est une pseudo-céréale comestible naturellement riche en phytoecdysones (Zhu et al., 2001 ; Dini et al., 2005). Il est ainsi possible de compléter l'alimentation par ingestion d'un extrait de quinoa enrichi en phytoecdysones, en introduisant cet extrait dans un aliment, tel qu'un produit laitier ou une boisson, ou en le consommant en tant que complément alimentaire, par exemple sous forme de gélules.

Le quinoa représente à ce jour la plante alimentaire la plus riche en phytoecdysones. Les graines de quinoa contiennent un mélange de phytoecdysones (Zhu et al., 2001). Ces phytoecdysones sont particulièrement abondantes dans l'enveloppe des graines de quinoa. Par exemple, une ration de graines de quinoa de 60 grammes (poids sec) apporte entre 15 et 25 milligrammes de 20-hydroxyecdysone.

Les phytoecdysones mises en oeuvre selon l'invention se présentent avantageusement sous la forme d'une composition pouvant être administrée par voie orale.

La composition s'entend par exemple d'un produit alimentaire tel qu'une boisson, un produit laitier ou autre. Bien entendu, la composition peut être une composition médicinale, par exemple utilisée sous forme de pilules qui contiennent ainsi une dose bien précise de phytoecdysones.

Avantageusement, les phytoecdysones sont administrées par voie orale à raison de 0,3 à 2,0 mg par Kg de masse corporelle et par jour, de préférence 0,5 mg par Kg et par jour.

Un autre objet de l'invention est un procédé de préparation d'un extrait de quinoa enrichi en une ou plusieurs phytoecdysones, ledit procédé comprenant les étapes suivantes :
- extraction à l'eau de graines de quinoa ;
- séparation solide/liquide et centrifugation de l'extrait aqueux ;
- chauffage du surnageant de sorte à précipiter des protéines ;
- purification par chromatographie du surnageant de sorte à l'enrichir en phytoecdysones.

L'invention se rapporte également à l'extrait de quinoa issu du procédé susmentionné. Cet extrait peut avantageusement être utilisé pour éviter la reprise de poids chez les mammifères obèses soumis à un régime hypocalorique amaigrissant, comme décrit ci-dessus.

### Brève description des figures

Figures 1A, 1B: Graphiques représentant l'évolution pondérale d'individus obèses soumis à un régime hypocalorique ;
- Figures 2A, 2B: Graphiques représentant l'évolution du diamètre adipocytaire d'individus obèses soumis à un régime hypocalorique ;
- Figures 3A, 3B: Graphiques représentant l'évolution de l'insulinémie d'individus obèses soumis à un régime hypocalorique ;
- Figures 4A, 4B: Graphiques représentant l'évolution de la sensibilité à l'insuline d'individus obèses soumis à un régime hypocalorique ;
- Figure 5 : Formules chimiques de phytoecdysones présentes dans une composition selon un mode de réalisation de l'invention.

### Description détaillée

Dans l'invention, on propose d'apporter une dose de phytoecdysones sous la forme de molécules purifiées, ou par le biais d'un extrait de plantes enrichi en phytoecdysones, afin d'éviter la reprise pondérale, ou « effet rebond », chez les mammifères obèses soumis à un régime hypocalorique amaigrissant.

Selon l'invention, il est possible d'apporter cette dose de phytoecdysones sous la forme d'un extrait de plantes, telles que de quinoa, incorporé par exemple dans un aliment entrant dans l'alimentation courante d'un individu. En effet, 1 gramme d'extrait de quinoa enrichi à hauteur de 2 % en poids de phytoecdysones, contient 20 milligrammes de phytoecdysones. Pour obtenir la même quantité de phytoecdysones à partir de graines de quinoa, il faudrait consommer de 50 grammes de graines de quinoa non traitées (Dini et al., 2005 ; Kumpun et al., 2011). L'extrait de quinoa selon l'invention peut ainsi contenir jusqu'à 50 fois plus de phytoecdysones que les graines de quinoa dont il est issu.

### I - Exemple de procédé de préparation d'extrait de quinoa enrichi en phytoecdysones (Extrait A)

Les graines de Quinoa sont tout d'abord moulues pour séparer la farine et le son de la graine. On procède à une extraction, en ajoutant 4.000 L d'eau à 400 Kg de son de graines. L'extrait aqueux subit une séparation solide/liquide suivie d'une centrifugation. Le surnageant ainsi obtenu est traité à la chaleur à 90°C pour précipiter les protéines. L'extrait aqueux est ensuite purifié par passage sur une colonne de résine alimentaire dans le but de l'enrichir en phytoecdysones. L'éluat ethanolique est ensuite séché par *spray drying* après ajout d'une quantité de maltodextrine, appropriée pour ajuster à la teneur de 2 ± 0,2 % en poids de 20-hydroxyecdysone (20E).

Une telle extraction séquentielle permet de supprimer de l'extrait les saponines, abondantes dans les graines de quinoa (Muir et al., 2002), qui conféreraient un goût amer audit extrait, ainsi que la majorité des sucres.

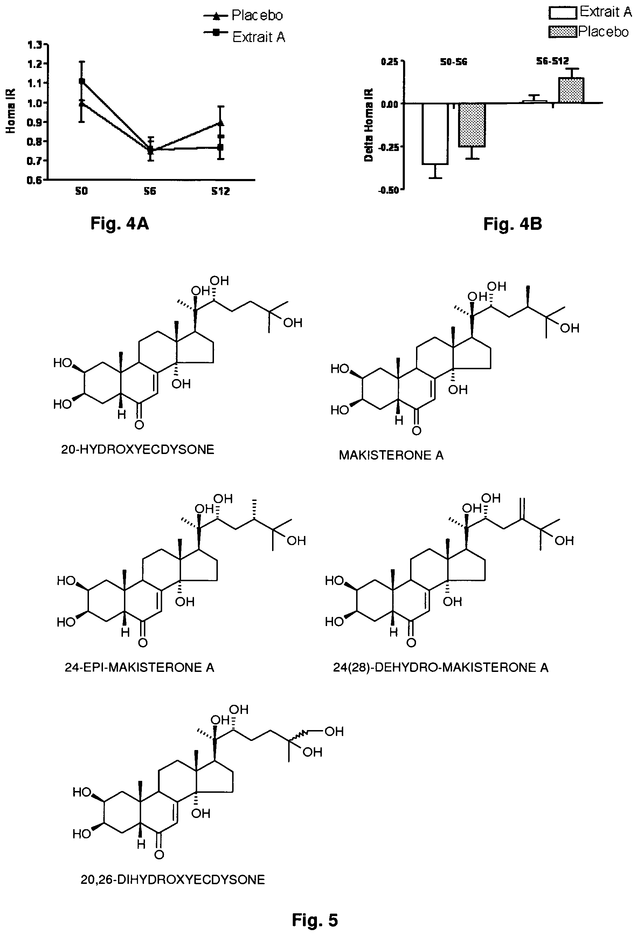

L'extrait obtenu contient un mélange de phytoecdysones dont 85-90 % correspondent à la 20-hydroxyecdysone, et le restant à d'autres phytoecdysones de structure très voisine, comme la makistérone A, la 24-épi-makistérone A, la 24(28)-déhydro-makistérone A ou la 20,26-dihydroxyecdysone (Kumpun et al., 2011). Les structures de ces composés sont représentées sur la figure 5.

Un extrait analogue à l'extrait A, utilisable dans le cadre de l'invention, est notamment commercialisé sous la marque Quinolia^{®}.

### Il - Etude clinique en double aveugle des effets de l'extrait A sur des individus obèses soumis à un régime hypocalorique pendant 6 semaines suivi d'un régime de stabilisation pendant 6 semaines

### Protocole

L'effet de l'extrait A a été étudié dans le cadre d'une étude clinique en double aveugle sur 60 volontaires en surpoids et obèses composés de 18 hommes et 42 femmes de BMI 27 à 38. Les volontaires sont soumis à un régime hypocalorique de 1200 Kcal chez les femmes et 1500 Kcal chez les hommes durant 6 semaines. Le régime hypocalorique est suivi d'un régime de stabilisation de 6 semaines, avec un apport calorique de 20% supérieur à celui du régime hypocalorique. Les paramètres tels que l'insulinémie, l'évolution pondérale, le diamètre adipocytaire et la force musculaire ont été mesurés lors des visites de début et de fin de phase de régime hypocalorique et de stabilisation.

Les sujets ont été répartis en deux groupes. Un premier groupe (groupe « extrait A ») a reçu 6 gélules d'extrait A contenant au total 40 mg de phytoecdysones, en 3 prises quotidiennes, durant toute la durée du test.

Un deuxième groupe témoin (groupe « placebo ») a reçu 6 gélules de placebo en 3 prises quotidiennes, pendant la même durée.

Les différentes mesures ont été effectuées en début de traitement (S0), au bout de la sixième semaine de régime (S6) et au bout de la douzième semaine de régime (S12).

### Mesure de l'évolution pondérale lors des régimes d'amaigrissement et de stabilisation

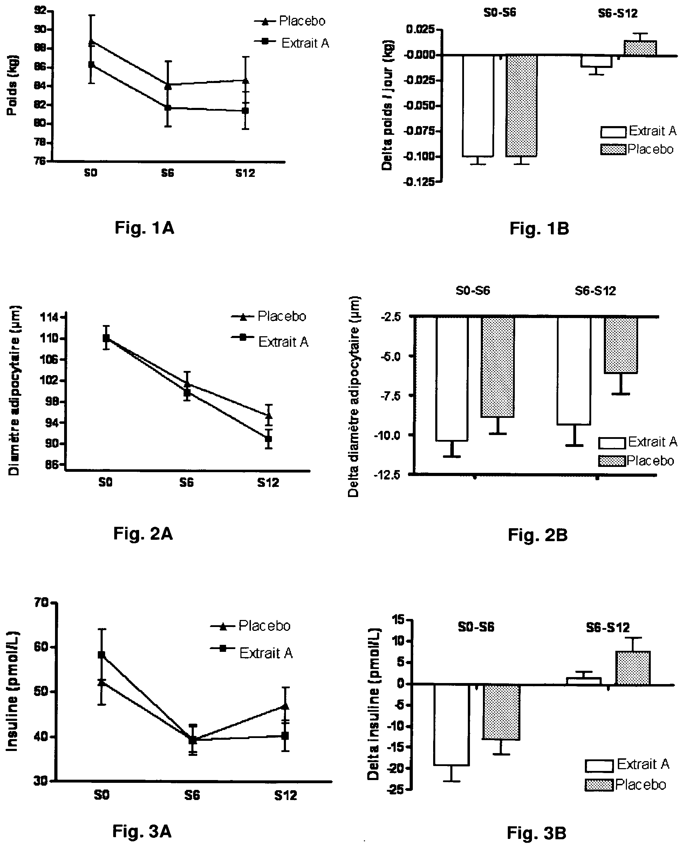

L'effet de l'extrait A lors d'un régime hypocalorique a été étudié sur l'évolution pondérale (Figures 1A et 1B).

Les groupes « placebo » et « extrait A » montrent des pertes de poids respectives de 4,05 et 3,86 kg lors de la première phase du régime, dite phase d'amaigrissement.

Lors de la deuxième phase du régime, dite phase de stabilisation, le groupe « extrait A » continue à perdre du poids (-0,483 kg) alors que le groupe « placebo » enregistre un gain moyen de +0,504 kg.

L'étude de l'évolution relative du poids des individus testés montre que le groupe « extrait A » ne présente que 10% d'individus ayant montré un rebond (gain de 0,05 à 0,1 kg/jour), valeur à comparer aux 28 % du groupe « placebo ».

Le même groupe « extrait A » présente 20% d'individus ayant perdu entre 0,06 et 0,15 kg/jour, alors que dans le groupe « placebo », cette catégorie ne représente que 10% des individus.

### Mesure du diamètre adipocytaire lors des régimes d'amaigrissement et de stabilisation

L'effet de l'extrait A lors d'un régime hypocalorique a été étudié sur l'évolution du diamètre adipocytaire (Figures 2A et 2B).

En phase d'amaigrissement, le diamètre adipocytaire moyen est réduit aussi bien dans le groupe « extrait A » (-10,94 µm) que dans le groupe « placebo » (-8.80 µm).

En phase de stabilisation, le diamètre moyen continue à diminuer, et on détecte une différence significative entre les groupes « extrait A » (-9,25 µm) et le groupe « placebo » (-5.99 µm).

Ce résultat est à relier à une perte de masse grasse supérieure en phase de stabilisation dans le groupe « extrait A ». En effet, la perte de masse grasse induite lors de la phase de stabilisation est de -0,74 kg pour le groupe « extrait A » alors que celle du groupe « placebo » n'est que de -0,33 kg.

### Mesure de l'évolution de l'insulinémie et de la résistance à l'insuline lors des régimes hypocalorique et de stabilisation

L'effet de l'extrait A a été étudié sur l'évolution de l'insulinémie (Figures 3A et 3B) et de la résistance à l'insuline (Figures 4A et 4B). L'indice HOMA-IR (*Homeostasis Model Assessment of Insulin Resistance*, Figures 4A et 4B) est un marqueur de la résistance à l'insuline.

L'insuline plasmatique diminue pendant la phase d'amaigrissement dans le groupe « extrait A » (-18,90 pmol/L) et le groupe « placebo » (-12,60 pmol/L). Celle-ci remonte fortement en phase de stabilisation dans le groupe « placebo » (7,69 pmol/L), alors qu'elle se stabilise dans le groupe « extrait A ». Le traitement à l'extrait A permet de stabiliser les taux d'insuline plasmatique dans la phase de stabilisation et de les diminuer significativement plus que le placebo tout au long du traitement.

La résistance à l'insuline, mesurée avec l'indice HOMA-IR, diminue dans les deux groupes pendant la phase d'amaigrissement. Elle reste constante dans le groupe « extrait A » durant la phase de stabilisation, alors qu'elle ré-augmente significativement dans le groupe « placebo ».

### Conclusions

L'administration de l'extrait A permet aux sujets obèses d'éviter une reprise pondérale durant la deuxième phase, dite phase de stabilisation, du régime hypocalorique amaigrissant.

Par ailleurs, l'administration de l'extrait A permet une diminution plus importante de la masse grasse et du diamètre adipocytaire moyen, lors de la phase de stabilisation.

L'administration de l'extrait A permet enfin de diminuer l'insulinémie et de maintenir lors de cette même phase l'amélioration de la sensibilité à l'insuline apportée par le régime hypocalorique.

### Références

Báthori M, Pongrácz Z. 2005. Phytoecdysteroids - from isolation to their effects on humans. Current Medicinal Chemistry 12, 153-172.
Dini I, Tenore GC, Dini A. 2005. Nutritional and antinutritional composition of Kancolla seeds: An interesting and underexploited andine food plant. Food Chemistry, 92, 125-132.
Flndeisen E. 2004 Ecdysteroids in Human food (Ecdysteroide in der menschlichen Nahrung). PhD thesis, University of Marburg, Germany.
Isomaa B, Almgren P, Tuomi T, Forsen B, Lahtii K, Nissen M, Taskinen MR, Groop L. 2001. Cardiovascular morbidity and mortality associated with the metabolic syndrome. Diabetes Care 24(4), 683-689.
Kumpun S, Maria A, Crouzet C, Evrard-Todeschi N, Girault JP, Lafont R. 2011. Ecdysteroids from Chenopodium quinoa Willd., an ancient Andean crop of high nutritional value, Food Chemistry 125, 1226-1234.
Kuzmenko Al, Niki E, Noguchi N. 2001. New functions of 20-hydroxyecdysone in lipid peroxidation. Journal of Oleo Science 50, 497-506.
McWorther LS. 2001. Biological complementary therapies: a focus on botanical products in diabetes. Diabetes Spectrum 14, 199-208.
Ogawa S, Nishimoto N, Matsuda H. 1974. Pharmacology of ecdysones in vertebrates. In: Invertebrate Endocrinology and Hormonal Heterophylly (Burdette, W. J. ed.) Springer, New York. 341-344.
Pittler MH, Schmidt K, Ernst E. 2005. Adverse events of herbal food supplements for body weight reduction : systematic review. Obesity Review 6, 93-111. Rexrode K, Carey V, Hennekens CH, Walters EE, Colditz GA, Stampfer MJ, Willett WC, Manson JE. 1998. Abdominal adiposity and coronary heart disease in women. JAMA 280, 1843-1848.
Saper RB, Eisenberg DM, Phillips RS. 2004. Common dietary supplements for weight loss. American Family Physician 70(9), 1731-1738.
Ulen CG, Huizinga M, Beech B, Elasy TA. 2008. Weight regain prevention. Clinical Diabetes 26(3), 100-113.
Zhu N, Kikusaki H, Vastano BC, Nakatani N, Karwe MV, Rosen RT, Ho CT. 2001. Ecdysteroids of quinoa seeds (Chenopodium quinoa Willd.). Journal of Agricultural and Food Chemistry 49, 2576-2578.

## Revendications

1. Phytoecdysones pour leur utilisation afin d'éviter la reprise pondérale chez les mammifères obèses après un régime hypocalorique amaigrissant.

2. Phytoecdysones pour leur utilisation selon la revendication 1, afin de stabiliser le diamètre adipocytaire et la masse grasse après un régime hypocalorique amaigrissant.

3. Phytoecdysones pour leur utilisation selon la revendication 1 ou la revendication 2, afin de stabiliser la sensibilité à l'insuline améliorée par un régime hypocalorique amaigrissant préalable.

4. Phytoecdysones pour leur utilisation selon l'une des revendications précédentes, telles qu'elles sont choisies parmi la 20-hydroxyecdysone, la makistérone A, la 24-épi-makistérone A, la 24(28)-déhydro-makistérone A, la 20,26-dihydroxyecdysone et les mélanges de deux ou plus de ces composés.

5. Phytoecdysones pour leur utilisation selon l'une des revendications précédentes, apportées sous forme d'un extrait de plantes.

6. Phytoecdysones pour leur utilisation selon la revendication 5, telles que l'extrait comporte au moins 1 % en masse de phytoecdysones.

7. Phytoecdysones pour leur utilisation selon l'une des revendications 5 à 6, telles que l'extrait de plantes provient du quinoa.

8. Phytoecdysones pour leur utilisation selon l'une des revendications précédentes, incorporées à une composition pouvant être administrée par voie orale.

9. Procédé de préparation d'un extrait de quinoa enrichi en une ou plusieurs phytoecdysones, ledit procédé comprenant les étapes suivantes :
- extraction à l'eau de graines de quinoa ;
- séparation solide/liquide et centrifugation de l'extrait aqueux ;
- chauffage du surnageant de sorte à précipiter les protéines ;
- purification par chromatographie du surnageant de sorte à l'enrichir en phytoecdysones.

## Patentansprüche

1. Phytoecdysone für die Verwendung zur Vermeidung einer erneuten Gewichtszunahme bei adipösen Säugern nach einer hypokalorischen Schlankheitsdiät.

2. Phytoecdysone für die Verwendung nach Anspruch 1, um den Adipozytendurchmesser und die Fettmasse nach einer hypokalorischen Schlankheitsdiät zu stabilisieren.

3. Phytoecdysone für die Verwendung nach Anspruch 1 oder Anspruch 2, um die durch eine vorherige hypokalorische Schlankheitsdiät verbesserte Insulinempfindlichkeit zu stabilisieren.

4. Phytoecdysone für die Verwendung nach einem der vorhergehenden Ansprüche, wie sie aus 20-Hydroxyecdyson, Makisteron A, 24-Epimakisteron A, 24(28)-Dehydromakisteron A, 20,26-Dihydroxyecdyson und den Gemischen von zweien oder mehreren dieser Verbindungen ausgewählt sind.

5. Phytoecdysone für die Verwendung nach einem der vorhergehenden Ansprüche, die in Form eines Pflanzenextrakts eingebracht werden.

6. Phytoecdysone für die Verwendung nach Anspruch 5, wobei der Extrakt mindestens 1%, bezogen auf die Masse, an Phytoecdysonen beinhaltet.

7. Phytoecdysone für die Verwendung nach einem der Ansprüche 5 bis 6, wobei der Pflanzenextrakt von Quinoa herrührt.

8. Phytoecdysone für die Verwendung nach einem der vorhergehenden Ansprüche, die in eine Zusammensetzung eingebracht werden, die auf oralem Weg verabreicht werden kann.

9. Verfahren zur Herstellung eines an einem oder mehreren Phytoecdysonen angereicherten Quinoaextrakts, wobei das Verfahren die folgenden Schritte umfasst:
- Extraktion von Quinoakörnern mit Wasser,
- Feststoff-/Flüssigkeits-Trennung und Zentrifugation des wässrigen Extrakts,
- Erhitzen des Überstands, um die Proteine auszufällen,
- Reinigung des Überstands durch Chromatographie, um ihn an Phytoecdysonen anzureichern.

## Claims

1. Phytoecdysones for their use in order to prevent weight regain in obese mammals after a hypocaloric weight-loss diet.

2. Phytoecdysones for their use according to claim 1, in order to stabilize the diameter of adipocytes and body fat after a hypocaloric weight-loss diet.

3. Phytoecdysones for their use according to claim 1 or claim 2, in order to stabilize the insulin sensitivity previously improved by a hypocaloric weight-loss diet.

4. Phytoecdysones for their use according to one of the previous claims, such that they are selected from 20-hydroxyecdysone, makisterone A, 24-epimakisterone A, 24(28)-dehydromakisterone A, 20,26-dihydroxyecdysone, and combinations of two or more of these components.

5. Phytoecdysones for their use according to one of the previous claims, provided in the form of a plant extract.

6. Phytoecdysones for their use according to claim 5, such that the extract comprises at least 1 % phytoecdysones by weight.

7. Phytoecdysones for their use according to one of claims 5 to 6, such that the plant extract comes from quinoa.

8. Phytoecdysones for their use according to one of the previous claims, incorporated into a composition that can be orally administered.

9. A method for preparing a quinoa extract enriched with one or more phytoecdysones, said method comprising the following steps:
- water extraction of quinoa seeds;
- solid/liquid separation and centrifugation of the aqueous extract;
- heating of the supernatant so as to precipitate proteins;
- purification by chromatography of the supernatant so as to enrich it with phytoecdysones.
